# EUROPEAN PATENT APPLICATION

(11) **EP 2 308 428 A1**
(43) Date of publication of application: **13.04.2011**
(21) Application number: 09172480.7
(22) Date of filing: 07.10.2009
(51) Int. Cl.: A61F 6/14, A61K 9/00

(54) **Device and method for reversible office sterilization**

(71) Applicant: Bio Material Systems, 9000 Ghent (BE); Wildemeersch, Dirk, 8301 Knokke-Heist (BE)
(72) Inventor: Wildemeersch, Dirk, 8301, Knokke-Heist (BE)
(74) Representative: Colens, Alain M.G.M.

(57) **Abstract**

The invention relates in the first place to a copper- releasing device (UTD) of extremely reduced dimensions and surface area to minimize the impact on menstrual blood loss. The device, consisting of hollow elements is constructed in a specific way to increase the lifespan of the UTD without reducing the exchange between the active substance of the device and the utero-tubal environment in order not to jeopardize the effectiveness of the contraceptive device.
Secondly, the invention relates also to a frameless utero-tubal hormone-releasing system (UTS), for contraception and treatment purposes, of reduced dimensions consisting of a coaxial, flexible rod, or multiple fibers, constructed in a specific way to increase the lifespan of the UTS.

## Description

### FIELD AND BACKGROUND OF THE INVENTION

Female tubal sterilization is the most common contraceptive method, used by one-fifth of married women worldwide. Around 200 million couples rely on the method for contraception. Twenty-one percent of married women in the world (13 % in more developed regions and 22 % in less developed countries) use tubal sterilization as their birth control method, compared with 4 % only of males using male sterilization (vasectomy). Methods of surgical sterilization vary: tubal sterilization by laparotomy or minilaparotomy, laparoscopic and hysteroscopic techniques of sterilization. In general, these methods are considered safe and effective. However, complications, although few, are not uncommon such as morbidity linked to surgery and anesthesia; and bowel and vascular injury in connection with laparoscopic procedures. It was calculated that, compared with vasectomy, tubal sterilization is 20 times more likely to have major complications, 10 to 37 times more likely to fail, and cost three times as much. Moreover, the procedure-related mortality, although rare, is 12 times higher with sterilization of a woman than of the man.

Hysteroscopic procedures are a new trend to avoid hospital admission, general anesthesia and to minimize complications. Essure™ is proposed as an office hysteroscopic procedure. However, although the Essure™ micro-insert sterilization method is considered an advance in female sterilization, in approximately 10% of women the implants cannot be properly placed during the first procedure. In a number of women (approximately 4%) in whom the inserts were properly placed, they were in an incorrect position at follow-up. Consequently, women cannot rely on the procedure, and have to use another contraceptive method until a hysterosalpingography has demonstrated bilateral tubal occlusion at follow-up. Failure rates during the first year of surgical and laparoscopic methods vary from 0.1 to 0.8%. At least one third of these are ectopic pregnancies. The CREST study identified a 10-year cumulative pregnancy rate of 18.5 failures per 1000 patients for all surgical and laparoscopic methods combined. With Essure™ clinical studies have shown the method to be 99.8% effective at 2 years of follow-up.

The Adiana™ method is another transcervical sterilization method which is applied in the office. The method consists of a combination of controlled thermal damage of the endosalpinx and insertion of a biocompatible matrix within the lumen of the oviduct. The method is irreversible. The clinical outcome from studies is similar to that of Essure™. It takes also several months until the woman can be assured about tubal occlusion.

Although sterilization is intended to be permanent, patient regret is not rare. Regret is often caused by unpredictable life conditions and occurs mainly in the younger age-groups. In a review study, regrets were expressed by over 20% of women aged 30 years or younger and by 6% of women older than 30 years. Therefore, the option of reversible method of sterilization might be attractive to many women. One study provided data which indicate that the availability of a reversible method of sterilization would significantly increase the acceptability of the method to potential candidates.

The presence of copper, high up in the uterine cavity, preferably close to the orifices of the fallopian tubes, causes a foreign body reaction widespread throughout the genital tract, including the fallopian tubes. Copper ions, together with products derived from the inflammatory reaction present in the luminal fluids of the genital tract, are toxic for spermatozoa and oocytes, preventing the encounter of healthy gametes and the formation of viable embryos. The current data do not indicate that embryos are formed in the presence of copper. The bulk of the data indicate that interference with the reproductive process after fertilization has taken place is exceptional in the presence of copper and that the usual mechanism by which pregnancy is prevented in women is by preventing fertilization.

Hormone-releasing intrauterine systems are well-known and have been developed for contraceptive purposes and for the treatment of various gynecological conditions such as heavy menstrual bleeding or menorrhagia. They act by suppressing the lining (endometrium) of the uterus so that sperm cannot reach the oviducts. These systems are therefore anti-fertility agents as they act prior to fertilization. If, however, an ovum would be fertilized, implantation will virtually be impossible due to the atrophic status of the endometrium caused by the local impact of the hormone released from the UTS (Utero Tubal System).

Copper tubes and drug delivery compartments can be permanently fixed in the fundus of the uterus between the orifices of the fallopian tubes. Such a fixation method was developed for the GyneFix^{®} IUD (Contrel Research, Ghent, Belgium). It has been shown in long-term clinical trials that the concept of suspending active ingredients in the uterus is a valid concept allowing permanent suspension until the device is removed simply by pulling on its lower end (tail). Upon insertion, the copper ions, released locally, will spread throughout the uterine cavity, preventing fertilization and ectopic pregnancy. The same happens with the released hormones. The risk of ectopic pregnancy is 90% lower than the risk among sexually active women using no contraception.

The present invention relates to new and improved "frameless" utero-tubal copper- and hormone-releasing intrauterine contraceptive devices and systems (UTD/UTS), remarkable well tolerated by any uterus due to the absence of a frame, and which have an extended lifespan.

The frameless characteristics of the current UTD (Utero Tubal Device) were first described in 1985 (priority application BE 214449; EP 0 191 747). However, at that time it was not known that the size of such a copper-releasing intrauterine device (IUD), could be reduced without affecting the effectiveness of the device. Further research found later that a reduced size had a significant impact on the amount of menstrual blood loss (MBL) due to the smaller the surface area of the device without reducing the effectiveness of the IUD. Additional in vitro research on GyneFix^{®} devices which were in place up to 12 years revealed the long duration of action of the GyneFix^{®} IUD. Previous dissolution studies of the GyneFix^{®} 330 IUD yielded a minimal dissolution rate, per copper tube, of 2.4 mg per year. This, in turn, confirms dissolution studies conducted with other copper IUDs which showed that copper is released constantly for long periods of time. These studies concluded that the estimation of the useful life-span of a copper-releasing IUD can be conducted on the basis of measurements of the copper weight, which is a linear, negative function of duration of use.

These studies concluded 1) that the copper release from the GyneFix^{®} IUD seems to be constant over a period up to 12 years; 2) that fragmentation of the copper tubes due to corrosion did not occur; 3) that the copper surface area did not decrease sufficiently to compromise contraceptive effectiveness; 4) that the "crimped" copper tubes last significantly longer than the "loose" copper tubes. The study suggests that the GyneFix^{®} IUD has a useful life-span of at least 10 years.

Fragmentation of copper is a major problem with copper IUDs which have a copper wire around the stem of the IUD. Due to corrosion, the wire can break and the copper can be lost resulting in absence or significant decreased pregnancy protection. The use of copper wire is, therefore, not useful for very long-term contraception. Only in the case of copper tubes is the risk of fragmentation of the copper and subsequent copper loss unlikely, even after long periods of time such as 12 years.

Similarily, in 1988, Belgian Priority Patent No 08801324 (corresponding to EP 0 445 150) described an improved intrauterine device which is a deformable element in the form of a plastic fiber releasing a substance which is active in the uterine cavity. The device carries a securing element for hooking it to the tissue of the uterus. The device was developed to be applicable for contraception and for treatment of the uterus.

The purpose of the present invention is to provide a frameless copper-releasing device but which lasts for 25 years instead of 12 years, and therefore, can be positioned as a reversible method of sterilization. Long duration of action is important for IUD users as it is economical and may reduce certain health risks related to frequent replacement. Due to the fact that the IUD consists of copper cylinders, the total weight of the copper can be increased thereby increasing the lifespan of the IUD by many years and at the same time reducing the risk of fragmentation of the copper which could increase the risk of pregnancy.

An additional purpose of the invention is to provide a frameless hormone-releasing system with the same long-acting characteristics as described above.

The invention is described as a reversible office sterilization procedure (ROS) which is a simple office procedure and usually can be performed without anesthesia (in women with children). It is inserted like GyneFix^{®} or it can be inserted using office hysteroscopy. The latter is the preferred mode of realization as it is conducted under direct vision by an experienced gynecologist. The amount of active substance in the copper-releasing UTD has been calculated to be sufficient to provide effective contraception for at least 25 years.

GyneFix^{®}, has shown in long-term clinical trials, conducted in collaboration with the World Health Organization, to have a superior effectiveness than the TCu380A IUD (Paragard^{®}, Duramed, Pomona, NY, USA). The effectiveness can be compared with the effectiveness of conventional irreversible sterilization methods. There are significantly less ectopic pregnancies with the GyneFix^{®} IUD than with TCu380A IUD which is attributed to the high position of the copper in the vicinity of the tubal ostia.

Similarly, the hormone-releasing UTS, releasing a strong progestogen, has been designed to last for over 20 years. A current levonorgestrel-releasing intrauterine system has a calculated lifespan of more than 10 years. Especially if stronger progestogens are used, an UTS lasting at least 25 years is currently being developed.

According to a preferred method of realization, ROS UTD/UTS is provided with tiny stainless steel tubes which are threaded onto the anchoring thread and is positioned immediately below the knot, and additionally onto the tail of the knot, which at insertion is implanted in the myometrium on both sides of the uterine fundus in the vicinity of the orifices of the fallopian tubes. These stainless steel tubes allow visibility on ultrasound examination and is a safeguard against perforation of the uterus. The rate of perforation at insertion is expected to be even lower due to the visualization of the anchor at the time of the procedure. Perforation of the uterine wall at a later date, a feared complication of intrauterine devices, on the other hand, will be impossible due to the design characteristics of the inventive double anchored UTD/UTS described hereafter.

### SUMMARY OF THE INVENTION

The present invention is directed to a small copper-releasing utero-tubal device (UTD), or to a hormone-releasing utero-tubal system (UTS), characterised in that it is fixed to the fundus of the uterus by means of two tiny anchors, securing the device between the orifices of the fallopian tubes. This is preferably performed by using "office hysteroscopy". The device has a lifespan of at least 20, preferably at least 25 years.

This purpose is also reached, according to one aspect of the present invention, by devising a device of very small dimensions, of which the active substance is copper, and of which the components are hollow and attached to each other one behind the other in a non-rigid assembly and of which the elements are sufficiently short allowing the inner surface of the cylinders to come into contact with the utero-tubal environment. The UTD/UTS is lying against the uppermost fundal wall of the uterus and, in case copper is used, consists of copper tubes. The surface area of the foreign body is low but the effective copper surface area is large, comparable with the effective copper surface area of the high-load conventional copper IUDs, and of which the total surface area of the foreign body is at least two or three times smaller than that of conventional copper releasing IUDs in order not to increase significantly the amount of menstrual blood loss.

The preferred mode of realization is to create a frameless UTD consisting of 4 copper tubes, each with outer diameter of 2.4 mm and an inner diameter of 1.6 mm, which are threaded on a length of suture material. The proximal end of the thread is provided with an anchoring means which serves as retaining member to keep the device affixed to the uterine wall.

According to another embodiment of the invention the upper and lower copper tubes are crimped onto the thread to prevent sliding off the suture thread.

According to another characteristic of the invention, the anchoring thread is provided with a stainless steel tube with inner and outer diameter slightly larger than the size of the anchoring thread allowing the stainless steel tube to be threaded onto the thread and to allow it to be grasped with a forceps to insert the anchor in the fundal muscle of the uterus.

According to yet another characteristic of the invention the anchor is made by heat-deformation. To this end, the thread should have at least a 0-gauge size to allow the formation of a body which is of sufficient size to properly function as an anchor when inserted in the wall of the uterus.

According to an additional characteristic of the invention, the copper consists of copper tubes, hollow on both sides.

According to still an additional characteristic of the invention, the outer diameter of the copper cylinders should not exceed 2.8 mm to allow easy insertion in the uterus.

According to an additional characteristic of the invention, the inner diameter of the copper cylinders should not be less than 1.6 mm to allow proper copper ion exchange with the uterine environment.

Another important characteristic of the invention is that the copper can be replaced by a drug delivery compartment, or by fibrous filaments, which have roughly the same surface area of that of the copper device.

### DESCRIPTION OF THE DRAWINGS

These and other characteristics of the invention will be more readily understood when referring to the description as well as the accompanying drawings which represent, merely by way of non-limitative examples, several embodiments of the invention, and in which:
FIG. 1 represents a view of a preferred embodiment of an UTD according to the invention,
FIG. 2 represents a view of a preferred embodiment of an UTS according to the invention,
FIG. 3 and 4 are schematic cross sectional views of the uterus with a preferred embodiment of an UTD according to the invention, showing the possibility of exchange of contact between the inner surface of the hollow cylinders and the utero-tubal fluid. The top and bottom hollow elements can be crimped onto the polypropylene thread, or not.
FIG. 5 represents an enlarged view of an anchor which is made by heat-deformation. A loop of the anchoring thread is heated until melting point to allow the two strings of the loop to melt into one solid, flattened spherical-shaped deformation, sufficiently large to allow secure anchoring of the UTD/UTS to the fundal wall of the uterus.
FIG. 6 represents the same enlarged view of the preferred anchor which is provided with a metal tube on the anchoring thread on which a very thin tube is threaded onto the filament to add sufficient stiffness to allow insertion of the anchor in the fundal muscle with a small forceps during hysteroscopy.
FIG. 7 represents the same enlarged view of the preferred anchor which is provided with a small metal tube on the anchoring string to allow visibility of the anchor during and after its insertion in the uterine muscle.

### DETAILED DESCRIPTION

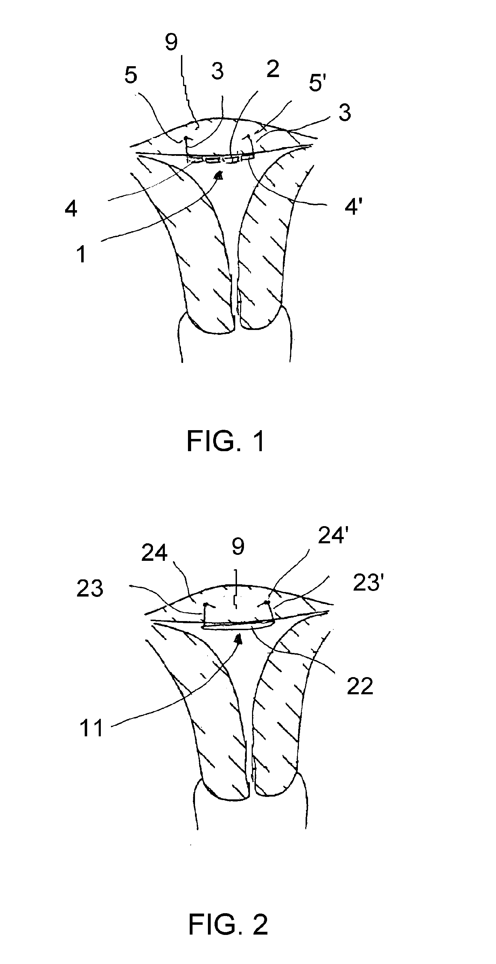

Referring now particularly to FIG. 1, an UTD 1 is illustrated in use in an uterus anchored to the fundul wall 9 of the uterus according to the invention. The device is made of copper which is active in the genital tract, open at both ends of the elements 2 arranged in a sequence in order to form a longitudinal body (tube or sleeve). The four elements are threaded on a length of polypropylene suture material 3. The tubes are loose (unfixed) onto the thread or the end sleeves 4 and 4', are crimped onto the thread. The proximal end of the thread is provided with two anchors 5 and 5', in the form of knot, to secure the UTD against the fundal wall of the uterus and prevent expulsion. This device has no rigid plastic body, making it a completely flexible unit.

According to the representation in FIG. 2, the UTS 11 is made of a drug delivery rod 22. The drug rod is affixed to an anchoring thread 23 and 23' on both extremities of the rod. The proximal end of the thread is provided with two anchors 24 and 24' to secure the UTS in the uterine cavity and prevent expulsion. This device is preferably a completely flexible unit.

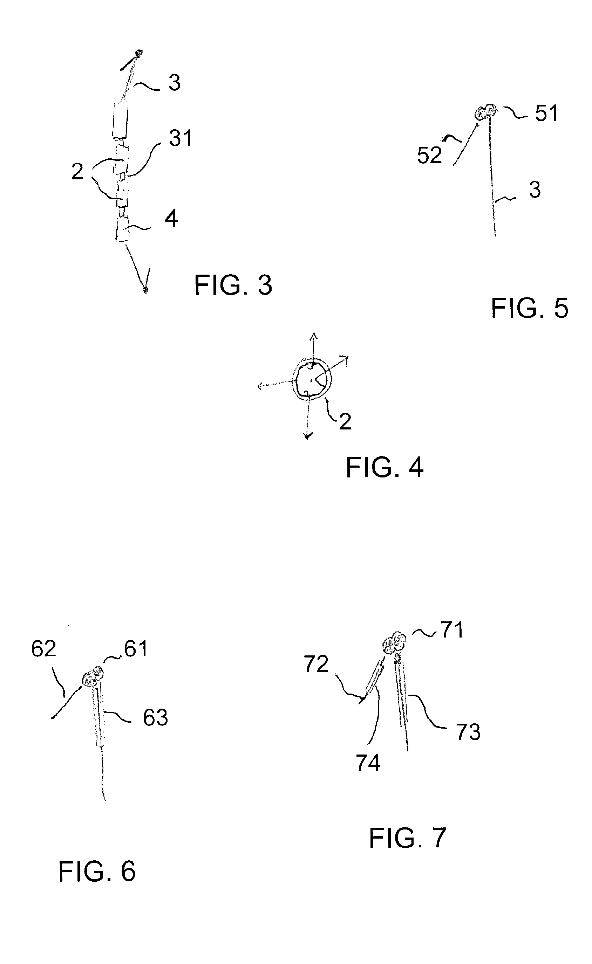

As shown in FIG. 3, which is a view according to one embodiment, the hollow elements, open at both ends, may be separated by a space, a spacer 31 being provided crimped onto the thread or not, to enhance the contact surface with the utero-tubal environment.

FIG. 4 is a sectional view of the hollow element 2 illustrating the exchange (arrows) between the inner surface of the hollow tube and the utero-tubal environment.

FIG. 5 is an enlarged view of the anchor consisting of the anchor body 51 with appendix 52 and anchoring thread 3. This assembly functions as the retaining body to suspend the active substance to the fundus of the uterus between the orifices of the oviducts.

FIG. 6 is an enlarged view of the anchor, according to the invention, of which the anchoring thread 61 is provided with a thin tube 62 which is threaded onto the anchoring thread up to the body of the anchor to allow the anchor to be inserted with a forceps or inserter into the musculature of the uterine fundus.

FIG. 7 is also an enlarged view of the anchor, according to the invention, of which the appendix of the anchor 71 is provided with a thin tube 72 which is threaded onto this portion of the anchor allowing still better visibility of the anchor at insertion.

It should be noted that the new anchoring systems described above may be applied in conjunction with known contraceptive devices, for example of the Gynefix type, where there is only one fixation point.

The technique for inserting the contraceptive device according to the invention is known and has already been described for non-rigid assembly for example in US 4,708,134. This technique makes use of an inserting device made of a needle and a protecting member.

The device is however preferably inserted into the uterine cavity firstly using an IUD inserter and, secondly using an office hysteroscope with a working channel of reduced dimension, both anchors are inserted into the uterine fundus using a (specially designed) grasping forceps introduced through the working channel of the hysteroscope.

The invention has been described and illustrated merely by way of examples which are in no way restrictive. Numerous changes in its conception may be made without departing from the spirit of the invention.

## Claims

1. A contraceptive device, active in the uterus as well as in the oviducts, comprising:
at least one metabolically active element, said device being arranged to form a longitudinal non-rigid structure, of limited dimensions compatible with the uterus cavity, **characterized in that** it comprises at each end of said structure a fixation means adapted to lie said device against the fundal wall of the uterus.

2. A contraceptive device according to claim 1 wherein the active element is a plurality of hollow cylinders stringed longitudinally on a thread to form the said non-rigid structure.

3. A contraceptive device according to claim 1 or 2 wherein the length of the structure, when the elements are closely approximated is less than 2.6 cm, and not more that 3.0 cm when the elements are separated along said thread, and/or the cylinder diameter is not more that 3.30 mm preferably not more than 3.0 mm; with an inner diameter of at least 1.6 mm, and/or wherein the total surface area does not exceed 275 mm2.

4. A contraceptive device according to any preceding claims wherein the fixation means is an anchor made by heat-deformation of the end of a loop made in an anchoring (thermoplastic) thread whereby the end is spherical and of which one string is cut short to form a hook, adding to the functionality of the anchor.

5. A contraceptive device according to any preceding claims **characterized in that** the hollow members are made of copper.

6. A contraceptive device according to any preceding claim **characterized in that** some of the hollow members are made of copper and the other(s) of gold and/or silver.

7. A contraceptive device according to any preceding claims **characterized in that** the hollow elements are kept in place by two small crimped clips, at the opposite ends of the sequence of the hollow elements, to maximize the effective copper surface area of the device.

8. A contraceptive device according to any preceding claims **characterized in that** at each end of the sequence the hollow elements are crimped onto a polypropylene thread to keep all hollow elements in place.

9. A contraceptive device according to claim 3 wherein the copper cylinders are at least 2.4 mm in diameter and maximum 2.8 mm; and wherein the wall thickness of each or part of the copper cylinders is at least 0.4 mm.

10. A contraceptive device according to any preceding claim **characterized in that** the total copper load is minimally 350 mg.

11. A contraceptive device according to any preceding claim, **characterized in that** the total length of the hollow members does not exceed 2.5 mm.

12. A contraceptive device according to any preceding claim **characterized in that** the effective copper surface area is between 220 mm² and 300 mm².

13. A contraceptive device according to claim 1 wherein the active element is a drug delivery compartment in the form of a rod of which the extremities are provided with fixation means, said fixation means comprising an anchoring thread and an anchor for insertion of said extremities in the uterine fundus and for the permanent suspension of the drug delivery rod to the fundus of the uterus.

14. A contraceptive device according to any preceding claim wherein the anchoring thread is provided with tiny tubes immediately below the anchoring knot to allow insertion of the anchor in the muscle of the uterine fundus.

15. A contraceptive device according to any preceding claim wherein the appendix of the anchor is provided with a tiny metal tube to allow visibility of the anchor during and after insertion in the uterine muscle.
